(19) Europäisches Patentamt / European Patent Office / Office européen des brevets

(11) EP 4 692 289 A1

(12) **EUROPEAN PATENT APPLICATION**
published in accordance with Art. 153(4) EPC

(43) Date of publication:
**11.02.2026 Bulletin 2026/07**

(21) Application number: **24779695.6**

(22) Date of filing: **19.03.2024**

(51) International Patent Classification (IPC):
***C11B 9/00*** *(2006.01)* ***C07C 29/141*** *(2006.01)*
***C07C 31/13*** *(2006.01)*

(52) Cooperative Patent Classification (CPC):
**C07C 29/141; C07C 31/13; C11B 9/00**

(86) International application number:
**PCT/JP2024/010640**

(87) International publication number:
**WO 2024/203570 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **31.03.2023 JP 2023057448**

(71) Applicant: **MITSUBISHI GAS CHEMICAL COMPANY, INC.**
**Chiyoda-ku**
**Tokyo 100-8324 (JP)**

(72) Inventors:
- **NAKAZAWA Yushun**
  **Kurashiki-shi, Okayama 712-8525 (JP)**
- **SHIRAI Shinyou**
  **Kurashiki-shi, Okayama 712-8525 (JP)**
- **HASHIMOTO Ryoma**
  **Kurashiki-shi, Okayama 712-8525 (JP)**

(74) Representative: **Vossius & Partner Patentanwälte Rechtsanwälte mbB**
**Siebertstrasse 3**
**81675 München (DE)**

# (54) PERFUME COMPOSITION AND ALICYCLIC ALCOHOL COMPOSITION

(57) A fragrance composition containing an alicyclic alcohol represented by Formula (1) below as an active ingredient. Provided are a fragrance composition containing, as an active ingredient, an alicyclic alcohol that can impart a smooth, clear and natural muguet scent to the fragrance composition, having a transparent, smooth, clear and natural muguet scent, and also having a clean green floral scent; use of the alicyclic alcohol as a fragrance; and an alicyclic alcohol composition containing the alicyclic alcohol and an isomer thereof.

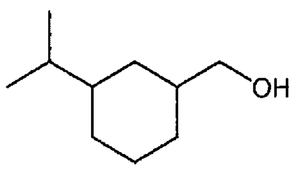

(1)

EP 4 692 289 A1

## Description

Technical Field

The present invention relates to a fragrance composition, use of an alicyclic alcohol as a fragrance, a method for producing an alicyclic alcohol, and an alicyclic alcohol composition.

Background Art

4-Isopropylcyclohexanemethanol (Mayol), which is an alicyclic alcohol, is a colorless liquid with a floral scent and is used as a raw material for floral and woody blended fragrances (Non-Patent Literature 1). In addition, as a production method, it is known that the compound can be obtained by reducing perillaldehyde and can be synthesized from β-pinene via perillaalcohol (Non-Patent Literature 1).

Citation List

Non-Patent Literature

Non-Patent Literature 1: Motoichi Indo, "Zoho Shin pan, Gousei Koryo: Kagaku to Shohin Chisiki (Enlarged and Revised Edition, Synthetic Fragrance: Chemistry and Product Knowledge)", The Chemical Daily Co. Ltd., 2016, pages 57 to 58

Summary of Invention

Technical Problem

In particular, floral fragrances and blended fragrances are used in various fields, such as fragrance products, cosmetics, detergents, hygiene products, sundries, pharmaceuticals, and food products. To increase the value of the products, new scents are being developed.

In particular, 4-isopropylcyclohexanemethanol has a floral scent as described above, but a fragrance having a new scent is in demand.

An object of the present invention is to provide a fragrance and a fragrance composition having a novel scent.

Solution to Problem

The present inventors have produced various alicyclic alcohols and alicyclic alcohol compositions, and evaluated their aromas, and found that a specific alicyclic alcohol and a specific alicyclic alcohol composition have an excellent scent, can be suitably used as fragrances, and, further, are excellent active ingredients of a fragrance composition, and completed the present invention.

That is, the present invention is as follows.

[1] A fragrance composition containing an alicyclic alcohol represented by Formula (1) below as an active ingredient:

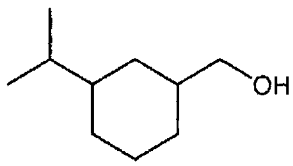
(1)

[2] The fragrance composition according to [1], further containing at least one selected from the group consisting of: a fragrance other than the alicyclic alcohol represented by Formula (1); a surfactant; a solvent; an antioxidant; and a colorant.

[3] Use of an alicyclic alcohol represented by Formula (1) below as a fragrance:

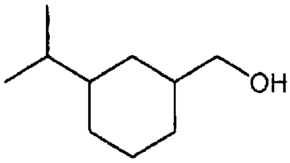 (1)

[4] A method for producing an alicyclic alcohol, the method including obtaining an alicyclic alcohol represented by Formula (1) below by hydrogenating an aromatic aldehyde represented by Formula (3) below:

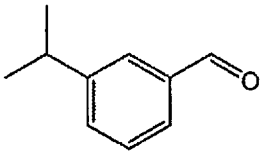 (3) 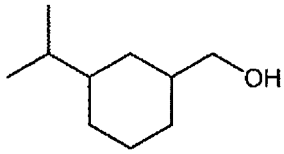 (1)

[5] An alicyclic alcohol composition containing an alicyclic alcohol represented by Formula (1) below and an alicyclic alcohol represented by Formula (2) below:

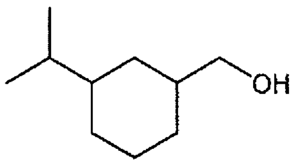 (1) 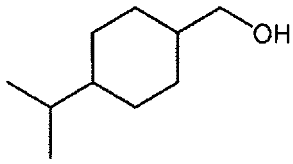 (2)

[6] The alicyclic alcohol composition according to [5], wherein a mass ratio [(1)/(2)] of the alicyclic alcohol represented by Formula (1) to the alicyclic alcohol represented by Formula (2) is from 78/22 to 99/1.

[7] A fragrance composition containing the alicyclic alcohol composition described in [5] or [6].

[8] The fragrance composition according to [7], further containing at least one selected from the group consisting of: a fragrance other than the alicyclic alcohol represented by Formula (1) or the alicyclic alcohol represented by Formula (2); a surfactant; a solvent; an antioxidant; and a colorant.

[9] Use of an aldehyde composition containing an alicyclic alcohol represented by Formula (1) below and an alicyclic alcohol represented by Formula (2) below as a fragrance:

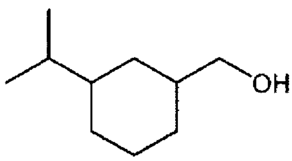 (1) 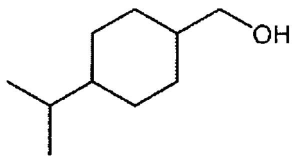 (2)

Advantageous Effects of Invention

According to the present invention, it is possible to provide a fragrance composition containing, as an active ingredient, an alicyclic alcohol that can impart a smooth, clear and natural muguet scent to the fragrance composition, having a transparent, smooth, clear and natural muguet scent, and also having a clean green floral scent; use of the alicyclic alcohol as a fragrance; and an alicyclic alcohol composition containing the alicyclic alcohol and an isomer thereof.

Description of Embodiments

Hereinafter, in the present description, the expression "from XX to YY" means "XX or more and YY or less", "XX or higher and YY or lower", or "XX or greater and YY or less".

[Fragrance composition containing alicyclic alcohol as active ingredient]

The fragrance composition of the present invention is a fragrance composition containing an alicyclic alcohol represented by Formula (1) below as an active ingredient. The compound represented by Formula (1) below is 3-isopropylcyclohexanemethanol.

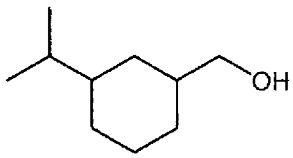 (1)

The fragrance composition of the present invention contains the alicyclic alcohol as an active ingredient. The alicyclic alcohol can impart a smooth, clear and natural muguet scent to the fragrance composition.

The fragrance composition of the present invention not only has the smooth, clear and natural muguet scent of the alicyclic alcohol but also has a transparent, smooth, clear and natural muguet scent and a clean green floral scent. Thus, the fragrance composition is useful as an aromatic component of various products.

A content of the alicyclic alcohol represented by Formula (1) in the fragrance composition of the present invention may be appropriately adjusted according to a type of fragrance composition, a type of target aroma, aroma intensity, and the like and is preferably from 0.01 to 90 mass%, more preferably from 0.1 to 50 mass%, even more preferably from 0.2 to 40 mass%, yet even more preferably from 0.5 to 30 mass%, yet even more preferably from 1 to 25 mass%, yet even more preferably from 3 to 20 mass%, and yet even more preferably from 4 to 15 mass%.

Examples of an additional component contained in the fragrance composition containing the alicyclic alcohol represented by Formula (1), in addition to the alicyclic alcohol represented by Formula (1), include a fragrance other than the alicyclic alcohol represented by Formula (1), a surfactant, a solvent, an antioxidant, and a colorants. The additional component is preferably at least one selected from the group consisting of a fragrance other than the alicyclic alcohol represented by Formula (1), a surfactant, a solvent, an antioxidant, and a colorant, and more preferably at least one selected from the group consisting of a fragrance other than the alicyclic alcohol represented by Formula (1) and a solvent.

The inclusion of a fragrance other than the alicyclic alcohol represented by Formula (1) allows the scent to be adjusted to accommodate a target product. In addition, the inclusion of a solvent allows for easy dissolution and impregnation into a target product, making it possible to adjust scent intensity or scent duration.

Among them, it is preferable to constitute a fragrance composition having at least one of a floral scent and a fruity scent, it is more preferable to constitute a fragrance composition having a floral fragrance, and it is even more preferable to constitute a fragrance composition having both a floral scent and a fruity scent, by a fragrance substance other than the alicyclic alcohol represented by Formula (1). The alicyclic alcohol represented by Formula (1) is preferably contained as an active ingredient of a fragrance composition having at least one of floral and fruity scents, more preferably contained as an active ingredient of a fragrance composition having a floral scent, and even more preferably contained as an active ingredient of a fragrance composition having both floral and fruity scents.

The fragrance other than the alicyclic alcohol represented by Formula (1) is not limited as long as it is a known fragrance component, and a wide range of fragrances can be used. For example, one, or two or more of fragrances described below can be selected and used at any mixing ratio.

Examples of the fragrance other than the alicyclic alcohol represented by Formula (1) include a hydrocarbon, an alcohol, a phenol, an ester, an aldehyde, a ketone, an acetal, a ketal, an ether, a nitrile, a lactone, a natural essential oil, and a natural extract, and preferably one or more fragrances selected from the group consisting of a hydrocarbon, an alcohol, a phenol, an ester, an aldehyde, a ketone, an acetal, a ketal, an ether, a nitrile, a lactone, a natural essential oil, and a natural extract. Among them, the fragrance other than the alicyclic alcohol represented by Formula (1) is preferably at least one selected from the group consisting of an aldehyde, a natural essential oil, a natural extract, an ether, and an ester, more preferably at least one selected from the group consisting of an ether and an ester, and even more preferably an ester.

Examples of the hydrocarbon include limonene, α-pinene, β-pinene, terpinene, cedrene, longifolene, and valencene.

Examples of the alcohol include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexanemethanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol. The alcohol is an alcohol other than the alicyclic alcohol represented by Formula (1).

Examples of the phenol include eugenol, thymol, and vanillin.

Examples of the ester include linalyl formate, citronellyl formate, geranyl formate, n-hexyl acetate, cis-3-hexenyl

acetate, linalyl acetate, citronellyl acetate, geranyl acetate, nellyl acetate, terpinyl acetate, nopil acetate, bornyl acetate, isobornyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate.

Examples of the aldehyde include n-octanal, n-decanal, n-dodecanal, 2-methylundecanal, 10-undecenal, citronellal, citral, hydroxycitronellal, dimethyl tetrahydrobenzaldehyde, 4(3)-(4-hydroxy-4-methylpentyl)-3-cyclohexene-1-carboaldehyde, 2-cyclohexyl propanal, p-t-butyl-α-methylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of the ketone include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohexedecenone.

Examples of the acetal and the ketal include acetoaldehyde ethylphenylpropyl acetal, citraldiethyl acetal, phenylacetoaldehyde glycerin acetal, and ethylacetoacetate ethylene glycol ketal.

Examples of the ether include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of the nitrile include citronellyl nitrile.

Examples of lactones include γ-nonalactone, γ-undecalactone, σ-decalactone, γ-jasmolactone, coumarin, cyclopentadecanolide, cyclohexadecanolide, ambrettolide, ethylene brassylate, and 11-oxahexadecanolide.

Examples of the natural essential oil and the natural extract include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the solvent include dipropylene glycol, diethyl phthalate, ethylene glycol, propylene glycol, methyl myristate, and triethyl citrate.

Examples of the surfactant include polyoxyethylene alkyl sulfate ether, and polyoxyethylene lauryl sulfate ether is preferable.

The fragrance composition containing the alicyclic alcohol represented by Formula (1) has a transparent, smooth, clear and natural muguet scent and also has a clean green floral scent, and therefore can be used as an aromatic component of various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor fragrances, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

An amount of the fragrance composition blended in the product described above is not particularly limited and can be selected depending on the type, nature, and sensory effect of the product to be perfumed. For example, the amount of the fragrance composition blended in the product is preferably 0.00001 mass% or more, more preferably 0.0001 mass% or more, and even more preferably 0.001 mass% or more. Also, the amount of the fragrance composition blended in the product is preferably 80 mass% or less, more preferably 60 mass% or less, and even more preferably 40 mass% or less.

Note that, when the fragrance composition is used as an aroma oil, perfume, or the like, the amount of the fragrance composition blended in the product may be 80 mass% or more, or may be 100 mass%.

[Use of alicyclic alcohol represented by Formula (1) as fragrance]

The alicyclic alcohol represented by Formula (1) below has a smooth, clear and natural muguet scent and can impart the smooth, clear, and natural muguet scent to a fragrance composition, and thus can be used as a fragrance. Use of an alicyclic alcohol represented by Formula (1) below as a fragrance is also included in the present invention.

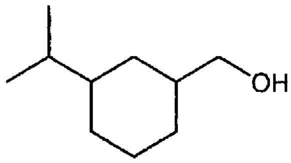

(1)

The compound represented by Formula (1) above is 3-isopropylcyclohexanemethanol.

[Method for producing alicyclic alcohol]

The method for producing the alicyclic alcohol represented by Formula (1) above is not limited, but the alicyclic alcohol is preferably obtained by a production method described below.

A preferable method for producing the alicyclic alcohol represented by Formula (1) above is a method of hydrogenating an aromatic aldehyde represented by Formula (3) below to obtain an alicyclic alcohol represented by Formula (1) below.

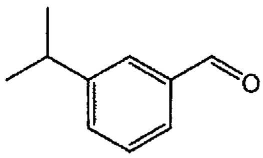

(3)

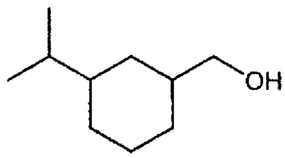

(1)

Here, the aromatic aldehyde represented by Formula (3) is 3-isopropylbenzaldehyde.

In the production method of the present invention, the hydrogenation method is not particularly limited. The hydrogenation can be performed by a known method using a hydrogenation catalyst.

The hydrogenation catalyst is not particularly limited, and a known catalyst can be used. The hydrogenation catalyst is preferably a supported heterogeneous hydrogenation catalyst, a Ziegler type hydrogenation catalyst, a homogeneous hydrogenation catalyst or a sponge metal catalyst.

The supported heterogeneous hydrogenation catalyst is a catalyst in which a metal such as Ni, Pt, Pd or Ru is supported on carbon, silica, alumina, diatomaceous earth or the like.

The Ziegler type hydrogenation catalyst uses a transition metal salt and a reducing agent such as organic aluminum. Examples of the transition metal salt include an organic acid salt or an acetyl acetone salt of Ni, Co, Fe, or Cr.

Examples of the homogeneous hydrogenation catalyst include an organometallic compound of Ti, Ru, Rh, or Zr.

Examples of the sponge metal catalyst include sponge nickel and sponge cobalt.

Temperature of a hydrogenation reaction in this step is preferably 0°C or higher, more preferably 10°C or higher, and even more preferably 20°C or higher, and is preferably 150°C or lower and more preferably 120°C or lower from the viewpoints of reactivity and suppressing of side reactions.

Pressure of hydrogen used in the hydrogenation reaction is preferably 0.01 MPaG or more, more preferably 0.03 MPaG or more, and even more preferably 0.05 MPaG or more, and is preferably 10 MPaG or less, more preferably 3 MPaG or less, even more preferably 1 MPaG or less, and yet even more preferably 0.5 MPaG or less.

Reaction time is not particularly limited but is preferably 3 minutes or more, more preferably 10 minutes or more, and even more preferably 30 minutes or more, and is preferably 24 hours or less, more preferably 12 hours or less, and even more preferably 8 hours or less.

The hydrogenation reaction may be performed in the presence of a solvent. The solvent to be used is not particularly limited as long as it does not inhibit the hydrogenation reaction, but is preferably a hydrocarbon solvent, and more preferably at least one selected from the group consisting of aliphatic hydrocarbons and alicyclic hydrocarbons.

Examples of the aliphatic hydrocarbon include pentane, hexane, isopentane, heptane, octane, and isooctane.

Examples of the alicyclic hydrocarbon include cyclopentane, methylcyclopentane, cyclohexane, methylcyclohexane, and ethylcyclohexane. One of these may be used individually, or two or more of these may be used in combination.

The method for purifying the target products, the alicyclic alcohol represented by Formula (1), from the solution after completion of the reaction is not particularly limited, and a known method may be appropriately selected and performed. Specifically, examples of the method include filtration, chromatography, and purification by distillation, and purification by appropriately combining these can provide a target alicyclic alcohol of high purity.

[Alicyclic alcohol composition]

An alicyclic alcohol composition of the present invention is an alicyclic alcohol composition containing an alicyclic alcohol represented by Formula (1) below and an alicyclic alcohol represented by Formula (2) below.

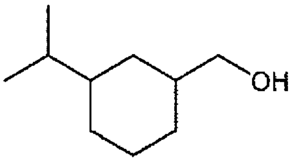 (1) 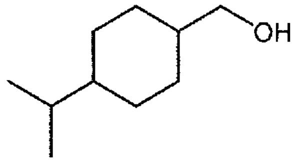 (2)

The alicyclic alcohol composition has a smooth and soft muguet scent and can impart the smooth and soft muguet scent to the fragrance composition. The alicyclic alcohol composition has softness particularly in a lasting scent.

The compound represented by Formula (1) above is 3-isopropylcyclohexanemethanol. The compound represented by Formula (2) above is 4-isopropylcyclohexanemethanol.

The alicyclic alcohol composition of the present invention contains the alicyclic alcohol represented by Formula (1) above and the alicyclic alcohol represented by Formula (2) above, and these two components alone may be used as components constituting the alicyclic alcohol composition of the present invention. However, in practice, by-products and raw materials produced when the alicyclic alcohol composition is produced may be contained.

Therefore, a total content of the alicyclic alcohol represented by Formula (1) above and the alicyclic alcohol represented by Formula (2) above in the alicyclic alcohol composition of the present invention is preferably 95 mass% or more, more preferably 96 mass% or more, and even more preferably 97 mass% or more. An upper limit of the total content is not limited, and may be 100 mass% or less, and the alicyclic alcohol composition of the present invention may consist only of the alicyclic alcohol represented by Formula (1) above and the alicyclic alcohol represented by Formula (2) above.

A mass ratio [(1)/(2)] of the alicyclic alcohol represented by Formula (1) above to the alicyclic alcohol represented by Formula (2) above in the alicyclic alcohol composition of the present invention is preferably from 78/22 to 99/1, more preferably from 78/22 to 95/5, and even more preferably from 78/22 to 90/10 from the viewpoints of smoothness and softness of the muguet scent, particularly from the viewpoint of softness of the lasting scent, and is yet even more preferably from 83/17 to 90/10 from the viewpoint of improving softness of the scent. In addition, particularly from the viewpoint of the softness of the lasting scent, the mass ratio is even more preferably from 83/17 to 95/5, and yet even more preferably from 83/17 to 90/10.

The alicyclic alcohol composition of the present invention is useful as a fragrance because it has a smooth and soft muguet scent. In particular, when the mass ratio of the alicyclic alcohol represented by Formula (1) above to the alicyclic alcohol represented by Formula (2) above is within the above range, the muguet scent increases, a very smooth and soft muguet scent is obtained along with a soft lasting scent, and thus the alicyclic alcohol composition becomes excellent as a fragrance. Furthermore, when incorporated into a fragrance composition, it can impart a very smooth and soft muguet scent to the fragrance composition.

[Use of alicyclic alcohol composition as fragrance]

An alicyclic alcohol composition containing an alicyclic alcohol represented by General Formula (1) below and an alicyclic alcohol represented by General Formula (2) below has a smooth and soft muguet scent and can impart the smooth and soft muguet scent to a fragrance composition, and thus can be used as a fragrance. The alicyclic alcohol composition can be suitably used as a fragrance because the composition has softness particularly in the lasting scent. Use of an alicyclic alcohol composition containing an alicyclic alcohol represented by General Formula (1) below and an alicyclic alcohol represented by General Formula (2) below as a fragrance is also included in the present invention.

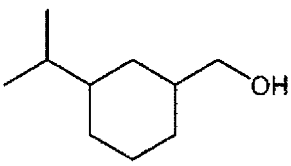 (1) 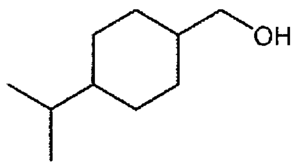 (2)

The compound represented by Formula (1) above is 3-isopropylcyclohexanemethanol. The compound represented by Formula (2) above is 4-isopropylcyclohexanemethanol.

A mass ratio [(1)/(2)] of the alicyclic alcohol represented by Formula (1) above to the alicyclic alcohol represented by Formula (2) above in the alicyclic alcohol composition used as a fragrance is preferably from 78/22 to 99/1, more preferably from 78/22 to 95/5, and even more preferably from 78/22 to 90/10 from the viewpoints of smoothness and

softness of the muguet scent, particularly from the viewpoint of softness of the lasting scent, and is yet even more preferably from 83/17 to 90/10 from the viewpoint of improving softness of the scent. In addition, particularly from the viewpoint of softness of the lasting scent, the mass ratio is even more preferably from 83/17 to 95/5, and yet even more preferably from 83/17 to 90/10.

The alicyclic alcohol composition used as a fragrance is useful as a fragrance because it has a smooth and soft muguet scent. In addition, it is useful as a fragrance because it has the soft lasting scent. In particular, when the mass ratio of the alicyclic alcohol represented by Formula (1) above to the alicyclic alcohol represented by Formula (2) above is within the above range, the muguet scent increases, a very smooth and soft muguet scent is obtained along with a soft lasting scent, and thus the alicyclic alcohol composition becomes excellent as a fragrance. Furthermore, when incorporated into a fragrance composition, it has a very smooth and soft muguet scent.

[Fragrance composition containing alicyclic alcohol composition]

The fragrance composition of the present invention contains the alicyclic alcohol composition.

That is, the fragrance composition of the present invention is a fragrance composition containing an alicyclic alcohol composition containing an alicyclic alcohol represented by Formula (1) below and an alicyclic alcohol represented by Formula (2) below.

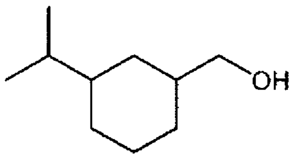 (1) 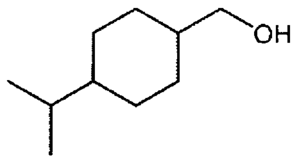 (2)

The fragrance composition of the present invention contains the alicyclic alcohol composition as an active ingredient. The alicyclic alcohol composition can impart a smooth and soft muguet scent to the fragrance composition.

The fragrance composition of the present invention has the smooth and soft muguet scent of the alicyclic alcohol composition. Thus, the fragrance composition is useful as an aromatic component of various products.

A mass ratio [(1)/(2)] of the alicyclic alcohol represented by Formula (1) above to the alicyclic alcohol represented by Formula (2) above in the alicyclic alcohol composition contained in the fragrance composition of the present invention is preferably from 78/22 to 99/1, more preferably from 78/22 to 95/5, and even more preferably from 78/22 to 90/10 from the viewpoints of smoothness and softness of the muguet scent, particularly from the viewpoint of softness of the lasting scent, and is yet even more preferably from 83/17 to 90/10 from the viewpoint of improving softness of the scent. In addition, particularly from the viewpoint of softness of the lasting scent, the mass ratio is even more preferably from 83/17 to 95/5, and yet even more preferably from 83/17 to 90/10.

The alicyclic alcohol composition contained in the fragrance composition of the present invention is useful as a fragrance because it has a smooth and soft muguet scent. In particular, when the mass ratio of the alicyclic alcohol represented by Formula (1) above to the alicyclic alcohol represented by Formula (2) above is within the above range, the muguet scent increases, a very smooth and soft muguet scent can be imparted to the fragrance composition.

The content of the alicyclic alcohol composition in the fragrance composition of the present invention may be appropriately adjusted according to the type of fragrance composition, the type of target aroma, the intensity of the aroma, and the like and is preferably from 0.01 to 90 mass%, more preferably from 0.1 to 50 mass%, even more preferably from 0.2 to 40 mass%, yet even more preferably from 0.5 to 30 mass%, yet even more preferably from 1 to 25 mass%, yet even more preferably from 3 to 20 mass%, and yet even more preferably from 4 to 15 mass%.

Examples of an additional component contained in the fragrance composition containing the alicyclic alcohol composition, in addition to the alicyclic alcohol composition, include fragrances other than the alicyclic alcohol represented by Formula (1) or the alicyclic alcohol represented by Formula (2), surfactants, solvents, antioxidants, and colorants. The additional component is preferably at least one selected from the group consisting of fragrances other than the alicyclic alcohol represented by Formula (1) or the alicyclic alcohol represented by Formula (2), surfactants, solvents, antioxidants, and colorants, and more preferably at least one selected from the group consisting of fragrances other than the alicyclic alcohol represented by Formula (1) or the alicyclic alcohol represented by Formula (2) and solvents.

Containing a fragrance other than the alicyclic alcohol represented by Formula (1) or the alicyclic alcohol represented by Formula (2) can adjust a scent according to a target product. In addition, the inclusion of a solvent allows for easy dissolution into and impregnation of a target product, making it possible to adjust the intensity of the scent or the durability of the scent.

Among them, it is preferable to constitute a fragrance composition having at least one of a floral scent and a fruity

scent, it is more preferable to constitute a fragrance composition having a floral fragrance, and it is even more preferable to constitute a fragrance composition having both a floral scent and a fruity scent, by a fragrance substance other than the alicyclic alcohol represented by Formula (1) and the alicyclic alcohol represented by Formula (2). The alicyclic alcohol composition is preferably contained as an active ingredient of a fragrance composition having at least one of floral and fruity scents, more preferably contained as an active ingredient of a fragrance composition having a floral scent, and even more preferably contained as an active ingredient of a fragrance composition having both floral and fruity scents.

The fragrance other than the alicyclic alcohol represented by Formula (1) or the alicyclic alcohol represented by Formula (2) is not limited as long as it is a known fragrance component, and a wide range of fragrances can be used. For example, one, or two or more of fragrances described below can be selected and used at any mixing ratio.

Examples of the fragrance other than the alicyclic alcohol represented by Formula (1) or the alicyclic alcohol represented by Formula (2) include hydrocarbons, alcohols, phenols, esters, aldehydes, ketones, acetals, ketals, ethers, nitriles, lactones, natural essential oils, and natural extracts, and preferably one or more fragrances selected from the group consisting of hydrocarbons, alcohols, phenols, esters, aldehydes, ketones, acetals, ketals, ethers, nitriles, lactones, natural essential oils, and natural extracts. Among them, the fragrance other than the alicyclic alcohol represented by Formula (1) and the alicyclic alcohol represented by Formula (2) is preferably at least one selected from the group consisting of aldehydes, natural essential oils, natural extracts, ethers, and esters, more preferably at least one selected from the group consisting of ethers and esters, and even more preferably an ester.

Examples of hydrocarbons include limonene, α-pinene, β-pinene, terpinene, cedrene, longifolene, and valencene.

Examples of alcohols include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol. The alcohol is an alcohol other than the alicyclic alcohol represented by Formula (1) or the alicyclic alcohol represented by Formula (2).

Examples of phenols include eugenol, thymol, and vanillin.

Examples of esters include linalyl formate, citronellyl formate, geranyl formate, n-hexyl acetate, cis-3-hexenyl acetate, linalyl acetate, citronellyl acetate, geranyl acetate, nellyl acetate, terpinyl acetate, nopil acetate, bornyl acetate, isobornyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate.

Examples of aldehydes include n-octanal, n-decanal, n-dodecanal, 2-methylundecanal, 10-undecenal, citronellal, citral, hydroxycitronellal, dimethyl tetrahydrobenzaldehyde, 4(3)-(4-hydroxy-4-methylpentyl)-3-cyclohexene-1-carboaldehyde, 2-cyclohexyl propanal, p-t-butyl-α-methylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of ketones include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohexedecenone.

Examples of acetals and ketals include acetoaldehyde ethylphenylpropyl acetal, citraldiethyl acetal, phenylacetoaldehyde glycerin acetal, and ethylacetoacetate ethylene glycol ketal.

Examples of ethers include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of nitriles include citronellyl nitrile.

Examples of lactones include γ-nonalactone, γ-undecalactone, σ-decalactone, γ-jasmolactone, coumarin, cyclopentadecanolide, cyclohexadecanolide, ambrettolide, ethylene brassylate, and 11-oxahexadecanolide.

Examples of natural essential oils and natural extracts include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the solvent include dipropylene glycol, diethyl phthalate, ethylene glycol, propylene glycol, methyl myristate, and triethyl citrate.

Examples of the surfactant include polyoxyethylene alkyl sulfate ether, and polyoxyethylene lauryl sulfate ether is

preferable.

The fragrance composition containing the alicyclic alcohol composition can be used as an aromatic component for various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor fragrances, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

The amount of the fragrance composition blended in the product described above is not particularly limited and can be selected depending on the type, nature, and sensory effect of the product to be perfumed. For example, the amount of the fragrance composition blended in the product is preferably 0.00001 mass% or more, more preferably 0.0001 mass% or more, and even more preferably 0.001 mass% or more. Also, the amount of the fragrance composition blended in the product is preferably 80 mass% or less, more preferably 60 mass% or less, and even more preferably 40 mass% or less.

Note that, when the fragrance composition is used as an aroma oil, perfume, or the like, the amount of the fragrance composition blended in the product may be 80 mass% or more, or may be 100 mass%.

[Method for producing alicyclic alcohol composition]

A method for producing an alicyclic alcohol composition containing the alicyclic alcohol represented by Formula (1) above and the alicyclic alcohol represented by Formula (2) above is not limited, but it is preferable to obtain the alicyclic alcohol composition by a production method described below.

Preferable methods for producing an alicyclic alcohol composition containing the alicyclic alcohol represented by Formula (1) above and the alicyclic alcohol represented by Formula (2) above include a method in which the alicyclic alcohol represented by Formula (1) above and the alicyclic alcohol represented by Formula (2) above are separately obtained and then mixed at a target ratio, and a method in which an aromatic aldehyde represented by Formula (3) below and an aromatic aldehyde represented by Formula (4) below are hydrogenated to obtain a mixture of an alicyclic alcohol represented by Formula (1) below and an alicyclic alcohol represented by Formula (2) below. Among them, the method in which an aromatic aldehyde represented by Formula (3) below and an aromatic aldehyde represented by Formula (4) below are hydrogenated to obtain a mixture of an alicyclic alcohol represented by Formula (1) below and an alicyclic alcohol represented by Formula (2) below is preferable. The method will be described below.

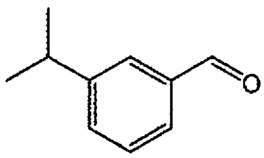 (3)

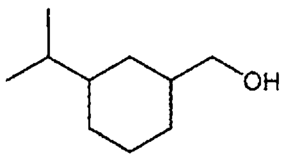 (1)

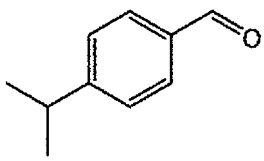 (4)

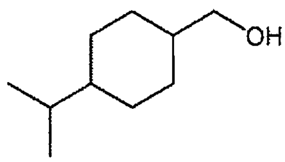 (2)

Here, the aromatic aldehyde represented by Formula (3) is 3-isopropylbenzaldehyde, and the aromatic aldehyde represented by Formula (4) is 4-isopropylbenzaldehyde.

In particular, when a raw material containing both the aromatic aldehyde represented by Formula (3) and the aromatic aldehyde represented by Formula (4) is used, a mass ratio [(3)/(4)] of the aromatic aldehyde represented by Formula (3) above to the aromatic aldehyde represented by Formula (4) above contained in the raw material of the present method is preferably from 78/22 to 99/1, more preferably from 78/22 to 95/5, and even more preferably from 78/22 to 90/10 from the viewpoints of smoothness and softness of the muguet scent, particularly from the viewpoint of softness of the lasting scent, and is yet even more preferably from 83/17 to 90/10 from the viewpoint of improving softness of the scent. In addition, particularly from the viewpoint of softness of the lasting scent, the mass ratio is even more preferably from 83/17 to 95/5, and yet even more preferably from 83/17 to 90/10.

When the mass ratio of the aromatic aldehyde represented by Formula (3) to the aromatic aldehyde represented

by Formula (4) is in the above range, an alicyclic alcohol composition having a target preferable scent can be obtained.

In the production method of the present invention, the hydrogenation method is not particularly limited. The hydrogenation can be performed by a known method using a hydrogenation catalyst.

The hydrogenation catalyst is not particularly limited, and a known catalyst can be used. The hydrogenation catalyst is preferably a supported heterogeneous hydrogenation catalyst, a Ziegler type hydrogenation catalyst, a homogeneous hydrogenation catalyst or a sponge metal catalyst.

The supported heterogeneous hydrogenation catalyst is a catalyst in which a metal such as Ni, Pt, Pd or Ru is supported on carbon, silica, alumina, diatomaceous earth or the like.

The Ziegler type hydrogenation catalyst uses a transition metal salt and a reducing agent such as organic aluminum. Examples of the transition metal salt include organic acid salts or acetyl acetone salts of Ni, Co, Fe, and Cr.

Examples of the homogeneous hydrogenation catalyst include organometallic compounds of Ti, Ru, Rh, and Zr.

Examples of the sponge metal catalyst include sponge metal catalysts such as sponge nickel and sponge cobalt.

The temperature of a hydrogenation reaction in this step is preferably 0°C or higher, more preferably 10°C or higher, and even more preferably 20°C or higher, and is preferably 150°C or lower and more preferably 120°C or lower from the viewpoints of reactivity and suppressing of side reactions.

The pressure of hydrogen used in the hydrogenation reaction is preferably 0.01 MPaG or more, more preferably 0.03 MPaG or more, and even more preferably 0.05 MPaG or more, and is preferably 10 MPaG or less, more preferably 3 MPaG or less, even more preferably 1 MPaG or less, and yet even more preferably 0.5 MPaG or less.

The reaction time is not particularly limited but is preferably 3 minutes or more, more preferably 10 minutes or more, and even more preferably 30 minutes or more, and is preferably 24 hours or less, more preferably 12 hours or less, and even more preferably 8 hours or less.

The hydrogenation reaction may be performed in the presence of a solvent. The solvent to be used is not particularly limited as long as it does not inhibit the hydrogenation reaction, but is preferably a hydrocarbon solvent, and more preferably at least one selected from the group consisting of aliphatic hydrocarbons and alicyclic hydrocarbons.

Examples of the aliphatic hydrocarbon include pentane, hexane, isopentane, heptane, octane, and isooctane.

Examples of the alicyclic hydrocarbon include cyclopentane, methylcyclopentane, cyclohexane, methylcyclohexane, and ethylcyclohexane. One of these may be used individually, or two or more of these may be used in combination.

The method for purifying the target product alicyclic alcohol composition or each alicyclic alcohol, from the solution after completion of the reaction is not particularly limited, and a known method may be appropriately selected and performed. Specifically, examples of the method include filtration, chromatography, and purification by distillation, and purification by appropriately combining these can provide a target alicyclic alcohol composition or each alicyclic alcohol of high purity. When the alicyclic alcohols are produced separately, they may be mixed to obtain the alicyclic alcohol composition.

## Examples

The present invention will be described in detail based on Examples presented below, but the present invention is not limited to these Examples.

### [Analysis and evaluation]

#### <Purity of alicyclic alcohol and composition ratio of alicyclic alcohol composition>

Purities and composition ratios of products obtained in examples and comparative examples were calculated and determined, according to an equation below, from the area of each peak in a chromatograph obtained by a gas chromatography analysis method under conditions described below. Note that the composition ratio ((1)/(2) ratio) of the alicyclic alcohol composition was calculated from a ratio of the proportions of the alicyclic alcohols in the alicyclic alcohol composition, the proportions being determined according to an equation below.

Purity (%) of alicyclic alcohol = peak area of target alicyclic alcohol/total area of all peaks excluding solvent × 100

Proportion (%) of target alicyclic alcohol (alicyclic alcohol represented by Formula (1) or alicyclic alcohol represented by Formula (2)) in alicyclic alcohol composition = peak area of target alicyclic alcohol/total peak area of alicyclic alcohol represented by Formula (1) and alicyclic alcohol represented by Formula (2) × 100

#### <Gas chromatographic analysis method (purity and composition ratio)>

Purities and composition ratios of products obtained in production examples and examples were determined by a

gas chromatographic method according to conditions described below.

Instrument used: Gas Chromatography Nexis GC-2030 (available from Shimadzu Corporation)
Column: DB-1 (30 m in length, 0.53 mm in inner diameter, and 1.5 μm in film thickness)
Detector: FID ($H_2$ 30 mL/min, Air 300 mL/min)
Carrier gas: He (constant flow; average linear velocity 38 cm/sec)
Split ratio: 28.1
Injection port temperature: 300°C
Detector temperature: 300°C
Injection volume: 1.0 μL
Oven temperature: The temperature was raised from 50°C to 150°C at 5°C/min, then raised to 280°C at 10°C/min after reaching 150°C, and then maintained for 7 minutes. The temperature was then raised to 300°C at 10°C/min and maintained for 5 minutes.

<GC-MS analysis method (structure of compound)>

GC-MS analysis was performed on products obtained in production examples and examples under conditions described below.

(GC Side)

Instrument used: Gas Chromatography GC2010 Plus (available from Shimadzu Corporation)
Column: DB-1MS (30 m in length, 0.25 mm in inner diameter, and 0.25 μm in film thickness)
Injection volume: 1 μL
Gasification chamber temperature: 300°C
Carrier gas: He
Linear velocity control: 38.0 cm/s
Split ratio: 28.1
Oven temperature: The temperature was raised from 50°C to 150°C at 5°C/min, then raised to 280°C at 10°C/min after reaching 150°C, and then maintained for 7 minutes. The temperature was then raised to 300°C at 10°C/min and maintained for 5 minutes.

(MS Side)

Instrument used: GCMS-QP2010 Ultra (available from Shimadzu Corporation)
Fragmentation method: EI
Ion source temperature: 200°C
Interface temperature: 250°C
Detector voltage: 0.8 kV

<NMR spectrum analysis method (structure of compound)>

Instrument: Varian NMR System PS600 600 MHz
Solvent: deuterated chloroform ($CDCl_3$)
Measurement mode: $^1H$, $^{13}C$
Internal standard substance: tetramethylsilane (TMS)

<Evaluation of scent/aromatic note>

The scent and aromatic note of alicyclic alcohols, alicyclic alcohol compositions and fragrance compositions of examples and comparative examples were evaluated by a method in which filter paper 8 mm in width and 15 cm in length was impregnated with the composition, and an expert panelist smelled the filter paper. Specific evaluation criteria and the

like are shown in the evaluation results of examples and comparative examples.

[Alicyclic alcohol represented by Formula (1)]

Example 1 (Production of 3-isopropylcyclohexanemethanol)

3-Isopropylbenzaldehyde (available from Fluorochem Ltd., 15 g), t-butyl alcohol (available from FUJIFILM Wako Pure Chemical Corporation, 160 g) and a 5% ruthenium-alumina catalyst (available from NE Chem Cat Corporation, dry product, powder type, 5 g) were charged into a 500-mL stainless steel autoclave, the inside of a reactor was replaced with nitrogen and hydrogen, hydrogen was then introduced so that the hydrogen pressure was 8.0 MPa, and a hydrogenation reaction was performed while stirring at a reaction temperature of 80°C for 6 hours. The reaction liquid was filtered to remove the catalyst, and then t-butyl alcohol was removed by simple distillation to obtain a crude composition.
The obtained crude composition was rectified at 10 torr using a rectification column having a theoretical plate number of 40 stages to obtain 3-isopropylcyclohexanemethanol (alicyclic alcohol represented by Formula (1)) as a fraction having a distillation temperature of 113°C.
Analytical results of 3-isopropylcyclohexanemethanol are shown below.

[Results of NMR spectrum measurement of 3-isopropylcyclohexanemethanol]

$^1$H NMR(600MHz, $CDCl_3$)$\delta$ 0.60-1.83(17H,m), 3.45(1H,t,J=8.4 Hz), 3.56(1H,t,J=8.4 Hz)

[Results of GC-MS measurement of 3-isopropylcyclohexanemethanol]

GC-MS m/z= 138(3), 125(6), 110(8), 95(100), 82(20), 69(40), 55(21)

[Alicyclic alcohol represented by Formula (2)]

Production Example 1 (Production of 4-isopropylcyclohexanemethanol)

4-Isopropylbenzaldehyde (available from Tokyo Chemical Industry Co., Ltd., 200 g) and a 5% ruthenium-alumina catalyst (available from NE Chem Cat Corporation, dry product, powder type, 10 g) were charged into a 500-mL stainless steel autoclave, the inside of a reactor was replaced with nitrogen and hydrogen, hydrogen was then introduced so that the hydrogen pressure was 8.0 MPa, and a hydrogenation reaction was performed while stirring at a reaction temperature of 80°C for 6 hours. The reaction liquid was filtered to remove the catalyst to obtain a crude composition.
The obtained crude composition was rectified at 8 torr using a rectification column having a theoretical plate number of 80 stages to obtain 4-isopropylhexanemethanol (alicyclic alcohol represented by Formula (2)) as a fraction having a distillation temperature of 108°C.
Analytical results of 4-isopropylcyclohexanemethanol are shown below.

[Results of NMR spectrum measurement of 4-isopropylcyclohexanemethanol]

$^1$H NMR(600 MHz, $CDCl_3$)$\delta$ 0.85-1.83(17H,m), 3.45(2H,t,J=14.4 Hz), 3.56(2H,t,J=14.4 Hz)

[Results of GC-MS measurement of 4-isopropylcyclohexanemethanol]

GC-MS m/z= 138(4), 125(6), 110(16), 95(100), 82(16), 69(26), 55(20)
The 3-isopropylcyclohexanemethanol obtained in Example 1 was evaluated for scent and aromatic note. The results are shown in Table 1. Further, the 4-isopropylcyclohexanemethanol obtained in Production Example 1 as Comparative Example 1 and FLOROL (isobutylmethyltetrahydropyranol) as Comparative Example 2 were also evaluated for scent and aromatic note. The results are shown in Table 1.
The scent and aromatic note were also evaluated based on Comparative Example 1. In addition, natural feeling, lasting scent properties, diffusibility, and alcohol odor were also evaluated. A compound having natural feeling, lasting scent properties and diffusibility is preferable as fragrance and fragrance composition component having a muguet scent, and a compound having no alcohol odor is preferable as fragrance and fragrance composition component having a muguet scent.
As aroma evaluation, the above evaluation results were comprehensively assessed according to criteria described below. A compound having more excellent natural feeling, lasting scent properties and diffusibility is a more excellent fragrance.

A: Having natural feeling, lasting scent properties, and diffusibility.
B: Having natural feeling, but not having one of lasting scent properties and diffusibility.
C: Having weak natural feeling (equivalent to Comparative Example 1).
D: Having no natural feeling.

[Table 1]

Table 1

| | Example 1 | Comparative Example 1 | Comparative Example 2 |
|---|---|---|---|
| | 3-Isopropylcyclohexanemethanol (purity: 99.9%) | 4-Isopropylcyclohexanemethanol (purity: 99.6%) | FLOROL (isobutylmethyltetrahydropyranol) (purity: 99.5%) |
| Evaluation of scent/aromatic note | Very smooth, clear and natural muguet. | Clear muguet. | Muguet. Woody feeling and alcohol solvent odor. |
| Comparison with Comparative Example 1 in evaluation of scent and aromatic note | Excellent natural feeling. | (Reference) | Negative woody feeling and alcohol solvent odor are observed. |
| Natural feeling | Present | Slightly present (weak) | Absent |
| Lasting scent properties | Present | Present | Slightly present (weak) |
| Diffusibility | Present | Present | Slightly present (weak) |
| Alcohol odor | Absent | Present | Present |
| Aroma evaluation | A | C | D |

The alicyclic alcohol of Example 1 had a smooth, clear and natural muguet scent. Further, it was excellent in diffusibility of the scent and lasting scent properties. Thus, the alicyclic alcohol of Example 1 can be used as a fragrance.

Example 2 and Comparative Examples 3 to 4 (green/floral/fruity fragrance composition)

As Example 2, the 3-isopropylcyclohexanemethanol (alicyclic alcohol represented by Formula (1)) obtained in Example 1 was added to the blended fragrance base (green/floral/fruity fragrance composition) shown in Table 2 so as to attain 5 mass%, and the scent and aromatic note were evaluated. As Comparative Example 3, FLOROL (isobutylmethyltetrahydropyranol) was added to the blended fragrance base shown in Table 2 so as to attain 5 mass%, and the scent and aromatic note were evaluated. Further, as Comparative Example 4, the amount of dipropylene glycol in the blended fragrance base shown in Table 2 was changed from 21.73 mass% to 26.73 mass%, and the scent and aromatic note were evaluated. In the evaluation of scent and aromatic note, natural feeling, woody odor, and alcohol odor were also evaluated. A composition having natural feeling is preferable as the green/floral/fruity fragrance composition, and a composition having neither a woody odor nor an alcohol odor is preferable as the green/floral/fruity fragrance composition.

[Table 2]

Table 2

| | Example 2 | Comparative Example 3 | Comparative Example 4 |
|---|---|---|---|
| Blended component | (parts by mass) | (parts by mass) | (parts by mass) |
| 3-Isopropylcyclohexylmethanol (Formula (1)) | 5 | - | - |
| FLOROL | - | 5 | - |
| Dipropylene glycol | 21.73 | 21.73 | 26.73 |
| Tetradecacylaldehyde | 0.1 | 0.1 | 0.1 |
| Amber One | 30 | 30 | 30 |
| Ambrettolide | 0.4 | 0.4 | 0.4 |
| Bergamot Rectified Art | 1 | 1 | 1 |
| Cedarwood Virginia | 2 | 2 | 2 |
| Cis-3-hexanol | 0.1 | 0.1 | 0.1 |
| Citral synthetic | 0.4 | 0.4 | 0.4 |
| Damascone delta | 0.2 | 0.2 | 0.2 |
| Dynascone 10 dipropylene glycol | 0.07 | 0.07 | 0.07 |
| Ethylene brassylate | 10 | 10 | 10 |
| Fenchyl acetate | 0.2 | 0.2 | 0.2 |
| Fruitate | 0.1 | 0.1 | 0.1 |
| Hedione | 20 | 20 | 20 |
| ISOANANAT | 0.02 | 0.02 | 0.02 |
| Lemon terpene | 3.5 | 3.5 | 3.5 |
| Liffarome | 0.1 | 0.1 | 0.1 |
| Ligustrol | 0.06 | 0.06 | 0.06 |
| Lime terpene | 1 | 1 | 1 |
| Methyl ionone gamma | 1 | 1 | 1 |
| Stemone | 0.02 | 0.02 | 0.02 |
| Terpineol | 1 | 1 | 1 |

(continued)

| | Example 2 | Comparative Example 3 | Comparative Example 4 |
|---|---|---|---|
| Blended component | (parts by mass) | (parts by mass) | (parts by mass) |
| Tetrahydridolinalool | 2 | 2 | 2 |
| Total | 100 | 100 | 100 |
| (Evaluation of scent/aromatic note) | | | |
| Natural feeling | Present | Absent | Absent |
| Woody odor | Absent | Present | Absent |
| Alcohol odor | Absent | Present | Absent |

As a result of the evaluation of scent and aromatic note, the fragrance composition of Example 2 had no rough tone, had a transparent natural muguet note, and had an increased clean green floral scent as compared with the fragrance compositions of the comparative examples. In addition, the fragrance composition had a very natural, transparent, deep and fresh muguet floral sensation (image of petals).

Example 3 and Comparative Examples 5 to 6 (marine/floral/fruity fragrance composition)

As Example 3, the 3-isopropylcyclohexanemethanol (alicyclic alcohol represented by Formula (1)) obtained in Example 1 was added to the blended fragrance base (marine/floral/fruity fragrance composition) shown in Table 3 so as to attain 10 mass%, and the scent and aromatic note were evaluated. As Comparative Example 5, FLOROL (isobutylmethyltetrahydropyranol) was added to the blended fragrance base shown in Table 3 so as to attain 10 mass%, and the scent and aromatic note were evaluated. Further, as Comparative Example 6, the amount of dipropylene glycol in the blended fragrance base shown in Table 3 was changed from 11.82 mass% to 21.82 mass%, and the scent and aromatic note were evaluated. In the evaluation of scent and aromatic note, natural feeling, woody odor, and alcohol odor were also evaluated. A composition having natural feeling is preferable as the marine/floral/fruity fragrance composition, and a composition having neither a woody odor nor an alcohol odor is preferable as the marine/floral/fruity fragrance composition.

[Table 3]

Table 3

| | Example 3 | Comparative Example 5 | Comparative Example 6 |
|---|---|---|---|
| Blended component | (parts by mass) | (parts by mass) | (parts by mass) |
| 3-Isopropylcyclohexylmethanol (Formula (1)) (alicyclic alcohol (Example 1)) | 10 | - | - |
| FLOROL | - | 10 | - |
| Dipropylene glycol | 11.82 | 11.82 | 21.82 |
| Tetradecacylaldehyde | 0.12 | 0.12 | 0.12 |
| Amber One | 5.2 | 5.2 | 5.2 |
| Ambroxide | 0.5 | 0.5 | 0.5 |
| Benzyl acetate | 1.2 | 1.2 | 1.2 |
| Boisambrene Forte | 0.4 | 0.4 | 0.4 |
| Anisylpropanal | 0.2 | 0.2 | 0.2 |
| Cis-3-hexynyl acetate | 0.04 | 0.04 | 0.04 |
| Cis-3-hexen-1-yl | 0.04 | 0.04 | 0.04 |

(continued)

| | Example 3 | Comparative Example 5 | Comparative Example 6 |
|---|---|---|---|
| Blended component | (parts by mass) | (parts by mass) | (parts by mass) |
| Citronellol | 0.7 | 0.7 | 0.7 |
| Damascenone | 0.1 | 0.1 | 0.1 |
| Decalactone gamma | 0.1 | 0.1 | 0.1 |
| Ethyllinalool | 3.6 | 3.6 | 3.6 |
| Ethylene brassylate | 8 | 8 | 8 |
| Floral ozone | 0.1 | 0.1 | 0.1 |
| Geraniol | 0.4 | 0.4 | 0.4 |
| Grapefruit oil | 4 | 4 | 4 |
| Hedione | 40 | 40 | 40 |
| Helional | 3 | 3 | 3 |
| Indole pure | 0.02 | 0.02 | 0.02 |
| Liffarome | 0.16 | 0.16 | 0.16 |
| Linalol acetate | 0.9 | 0.9 | 0.9 |
| Magnolan | 0.8 | 0.8 | 0.8 |
| Mandarinal 32048 SAEF | 0.1 | 0.1 | 0.1 |
| Musk 50 IPM | 8.5 | 8.5 | 8.5 |
| Total | 100 | 100 | 100 |
| (Evaluation of scent/aromatic note) | | | |
| Natural feeling | Present | Absent | Absent |
| Woody feeling | Absent | Present | Absent |
| Alcohol odor | Absent | Present | Absent |

As a result of the evaluation of scent and aromatic note, the fragrance composition of Example 3 had no rough tone, had a transparent natural muguet note, and had an increased clean green floral scent as compared with the fragrance compositions of the comparative examples. Furthermore, the fragrance composition of Example 3 had a light, natural and sweet muguet scent with high palatability.

From the results of Tables 2 and 3, it can be seen that the fragrance composition of the present invention has a transparent and natural muguet scent because it contains the alicyclic alcohol as an active ingredient. It can be seen that the fragrance composition has a clean green floral scent as a whole.

[Alicyclic alcohol composition]

Examples 4 to 10 (Production of alicyclic alcohol composition)

The 3-isopropylcyclohexanemethanol (alicyclic alcohol represented by Formula (1)) obtained in Example 1 and the 4-isopropylcyclohexanemethanol (alicyclic alcohol represented by Formula (2)) obtained in Production Example 1 were mixed in proportions (mass%) shown in Table 4 to obtain alicyclic alcohol compositions of Examples 4 to 10.

The alicyclic alcohol compositions obtained in Examples 4 to 10 were evaluated for scent and aromatic note. The results are shown in Table 4. The 3-isopropylhexanemethanol (alicyclic alcohol represented by Formula (1)) obtained in Example 1 and the 4-isopropylhexanemethanol (alicyclic alcohol represented by Formula (2)) obtained in Production Example 1 (referred to as Comparative Example 1 in the same manner as in Table 1) were also evaluated for scent and aromatic note. The results are shown in Table 4.

All the alicyclic alcohol compositions and alicyclic alcohols had a muguet scent.

In the evaluation of scent and aromatic note, the muguet scent was evaluated in terms of smoothness and softness and softness of the lasting scent. Those having smoothness and softness and softness of the lasting scent are

preferred as fragrances, and are also preferred as fragrance composition components.

[Table 4]

Table 4

| | Example 1 | Example 4 | Example 5 | Example 6 | Example 7 | Example 8 | Example 9 | Example 10 | Comparative Example 1 (Production Example 1) |
|---|---|---|---|---|---|---|---|---|---|
| 3-Isopropylcyclohexylmethanol (Formula (1)) | 100 | 99 | 95 | 90 | 87.5 | 85 | 82.5 | 80 | 0 |
| 4-Isopropylcyclohexylmethanol (Formula (2)) | 0 | 1 | 5 | 10 | 12.5 | 15 | 17.5 | 20 | 100 |
| (Evaluation of scent) | | | | | | | | | |
| Smoothness/softness | Slightly present (weak) | Slightly present (weak) | Slightly present (weak) | Present | Considerably present (strong) | Present | Slightly present (weak) | Slightly present (weak) | Insufficient |
| Softness of lasting scent | Insufficient | Slightly present | Present | Present | Considerably present (strong) | Present | Slightly present (weak) | Slightly present (weak) | Insufficient |

The alicyclic alcohol compositions of the examples had a very smooth and soft muguet scent. Further, the alicyclic alcohol compositions of the examples had softness in the lasting scent. Therefore, the alicyclic alcohol compositions of the examples can be used as fragrances, and can also be suitably used as active ingredients of fragrance compositions.

## Claims

1. A fragrance composition comprising an alicyclic alcohol represented by Formula (1) as an active ingredient:

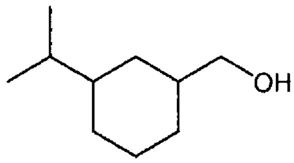 (1)

.

2. The fragrance composition according to claim 1, further comprising at least one selected from the group consisting of: a fragrance other than the alicyclic alcohol represented by Formula (1); a surfactant; a solvent; an antioxidant; and a colorant.

3. Use of an alicyclic alcohol represented by Formula (1) as a fragrance:

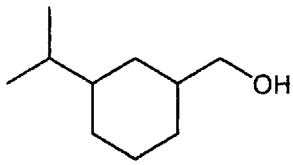 (1)

.

4. A method for producing an alicyclic alcohol, the method comprising obtaining an alicyclic alcohol represented by Formula (1) by hydrogenating an aromatic aldehyde represented by Formula (3):

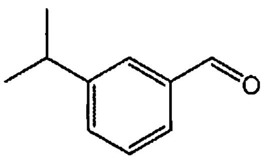 (3) 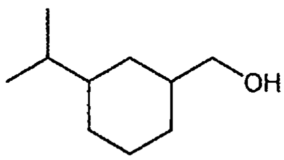 (1)

.

5. An alicyclic alcohol composition comprising an alicyclic alcohol represented by Formula (1) and an alicyclic alcohol represented by Formula (2):

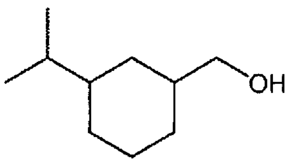 (1) 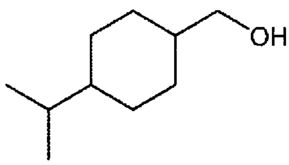 (2)

.

6. The alicyclic alcohol composition according to claim 5, wherein a mass ratio [(1)/(2)] of the alicyclic alcohol represented by Formula (1) to the alicyclic alcohol represented by Formula (2) is from 78/22 to 99/1.

7. A fragrance composition comprising the alicyclic alcohol composition described in claim 5 or 6.

8. The fragrance composition according to claim 7, further comprising at least one selected from the group consisting of: a fragrance other than the alicyclic alcohol represented by Formula (1) or the alicyclic alcohol represented by Formula

(2); a surfactant; a solvent; an antioxidant; and a colorant.

**9.** Use of an aldehyde composition comprising an alicyclic alcohol represented by Formula (1) and an alicyclic alcohol represented by Formula (2) as a fragrance:

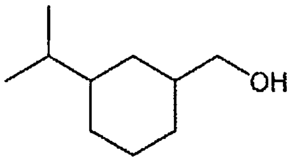 (1) 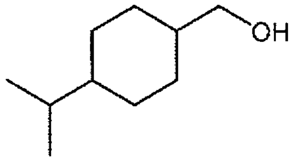 (2).

**INTERNATIONAL SEARCH REPORT**

International application No. **PCT/JP2024/010640**

**A. CLASSIFICATION OF SUBJECT MATTER**

***C11B 9/00***(2006.01)i; ***C07C 29/141***(2006.01)i; ***C07C 31/13***(2006.01)i
FI: C11B9/00 D; C07C31/13; C07C29/141

According to International Patent Classification (IPC) or to both national classification and IPC

**B. FIELDS SEARCHED**

Minimum documentation searched (classification system followed by classification symbols)

C11B9/00; C07C29/141; C07C31/13

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | RN 1697931-76-2 3-(1-Methylethyl)cyclohexanemethanol, Database Registry [online], CHEMICAL ABSTRACT SERVICE, US; [retrieved on 02 April 2024], retrieved from STN, entered STN: 04 May 2015<br>particularly, 3-(1-Methylethyl)cyclohexanemethanol | 1-3, 9 |
| Y | particularly, 3-(1-Methylethyl)cyclohexanemethanol | 4-8 |
| A | US 4847425 A (BASF AKTIENGESELLSCHAFT) 11 July 1989 (1989-07-11)<br>claims, examples | 1-3, 7-9 |
| Y | claims, examples | 4 |
| A | JP 08-311075 A (HAARMANN & REIMER GMBH) 26 November 1996 (1996-11-26)<br>claims, table 1, examples, etc. | 1-3, 9 |
| Y | claims, table 1, examples, etc. | 5-8 |
| A | JP 50-035351 A (FIRMENICH SA) 04 April 1975 (1975-04-04)<br>claims, examples, etc. | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 May 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/010640**

**C. DOCUMENTS CONSIDERED TO BE RELEVANT**

| Category* | Citation of document, with indication, where appropriate, of the relevant passages | Relevant to claim No. |
|---|---|---|
| A | ESCHER, S. D. et al., A quantitative study of factors that influence the substantivity of fragrance chemicals on laundered and dried fabrics, Journal of the American Oil Chemists' Society. (1994), vol. 71, no. 1, pp. 31-40, DOI 10.1007/BF02541469<br>abstract, etc. | 1-9 |
| A | RASTOGI, S. C. et al., Fragrances and other materials in deodorants: search for potentiall y sensitizing molecules using combined GC-MS and structure activity relationship (SAR) analysis. Contact Dermatitis, (1998), vol. 39, no. 6, pp. 293-303, DOI 10.1111/j.1600-0536.19 98.tb05944.x<br>tables 1, 2 | 1-9 |
| A | JP 48-098012 A (UNILEVER N.V.) 13 December 1973 (1973-12-13)<br>claims, etc. | 1-9 |
| A | JP 52-072826 A (MONSANTO CO.) 17 June 1977 (1977-06-17)<br>claims, etc. | 1-9 |
| A | JP 05-295388 A (KANEBO LTD.) 09 November 1993 (1993-11-09)<br>claims, etc. | 1-9 |

Form PCT/ISA/210 (second sheet) (July 2022)

**INTERNATIONAL SEARCH REPORT**

International application No.
**PCT/JP2024/010640**

**Box No. III Observations where unity of invention is lacking (Continuation of item 3 of first sheet)**

This International Searching Authority found multiple inventions in this international application, as follows:

(Invention 1) Claims 1-3

Claims 1-2 have the special technical feature of a "fragrance composition comprising, as an active ingredient, 1-ethyl cyclohexanecarboxylate ester represented by general formula (1)", and thus are classified as invention 1.

Claim 3 is related to claim 1 and thus is classified as invention 1.

(Invention 2) Claim 4

Claim 4 is the invention pertaining to a method for producing an alicyclic alcohol represented by formula (1), comprising hydrogenizing an aromatic aldehyde represented by formula (3) to obtain the alicyclic alcohol, and is not considered to share a same or corresponding special technical feature with claims 1-3 classified as invention 1.

In addition, claim 4 is not dependent on any of claims 1-3 and is not substantially identical to or similarly closely related to any of the claims classified as invention 1.

Claim 4 therefore cannot be classified as invention 1 and thus is classified as invention 2.

(Invention 3) Claims 5-9

Claims 5-8 is the invention pertaining to an alicyclic alcohol composition, comprising an alicyclic alcohol represented by formula (1) and an alicyclic alcohol represented by formula (2), and are not considered to share a same or corresponding special technical feature with claims 1-3 classified as invention 1, or are not considered to share a same or corresponding special technical feature with claim 4 classified as invention 2.

In addition, claims 5-9 are not dependent on any of claims 1-4, and are not substantially identical to or similarly closely related to any of the claims classified as invention 1 or 2.

Claims 5-9 therefore cannot be classified as invention 1 or 2 and thus are classified as invention 3.

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/010640**

| Patent document cited in search report | Publication date (day/month/year) | Patent family member(s) | Publication date (day/month/year) |
| --- | --- | --- | --- |
| US 4847425 A | 11 July 1989 | EP 293739 A1 | |
| JP 08-311075 A | 26 November 1996 | EP 742220 A2<br><br>DE 19517504 A1 | |
| JP 50-035351 A | 04 April 1975 | US 3993604 A<br>claims, examples<br>GB 1416658 A<br>DE 2427609 A1<br>FR 2232533 A1<br>CH 578312 A5 | |
| JP 48-098012 A | 13 December 1973 | US 4029759 A<br>claims<br>GB 1422272 A<br>DE 2309256 A1<br>FR 2174104 A1<br>CH 578841 A5 | |
| JP 52-072826 A | 17 June 1977 | US 4622221 A<br>claims, examples<br>GB 1545562 A<br>DE 2656405 A1<br>FR 2335244 A1 | |
| JP 05-295388 A | 09 November 1993 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MOTOICHI INDO**. Zoho Shin pan, Gousei Koryo: Kagaku to Shohin Chisiki (Enlarged and Revised Edition, Synthetic Fragrance: Chemistry and Product Knowledge). The Chemical Daily Co. Ltd., 2016, 57-58